(19) Europäisches Patentamt
European Patent Office
Office européen des brevets

(11) Publication number: 0 448 811 A1

# (12) EUROPEAN PATENT APPLICATION

(21) Application number: 90124579.5

(51) Int. Cl.5: C07K 5/00, A61K 37/02

(22) Date of filing: 18.12.90

The applicant has subsequently filed a sequence listing and declared, that it includes no new matter.

(30) Priority: 21.12.89 IT 2779189

(43) Date of publication of application: 02.10.91 Bulletin 91/40

(84) Designated Contracting States: AT BE CH DE DK ES FR GB GR IT LI LU NL SE

(71) Applicant: **SCLAVO S.p.A.**
**Via Fiorentina 1**
**I-53123 Siena(IT)**

(72) Inventor: **Becherucci, Cristina**
**Via Martiri di Niccioleta, 5**
**I-58037 Santa Fiora (Prov. of Grosseto)(IT)**
Inventor: **Perretti, Mauro**
**Via Montebello, 35**
**I-50123 Florence(IT)**
Inventor: **Silvestri, Sergio**
**Via Molizia, 59**
**I-53100 Siena(IT)**
Inventor: **Parente, Luca**
**Via Fracassi, 20**
**I-53100 Siena(IT)**

(74) Representative: **Gervasi, Gemma et al**
**NOTARBARTOLO & GERVASI Srl Viale Bianca Maria 33**
**I-20122 Milan(IT)**

## (54) New pharmaceutical composition with anti-inflammatory activity.

(57) The use of natural tuftsin (I) and retro-inverted tuftsin (II) or a pharmaceutically acceptable salt of basic or acid addition thereof as anti-inflammatory agents for the treatment of illnesses of inflammatory pathogenesis is described, together with pharmaceutical compositions comprising a therapeutically effective quantity of compound (I) or (II) or of their salts of addition.

EP 0 448 811 A1

## NEW PHARMACEUTICAL COMPOSITION WITH ANTI-INFLAMMATORY ACTIVITY

This invention relates to the use of natural tuftsin of formula (I):

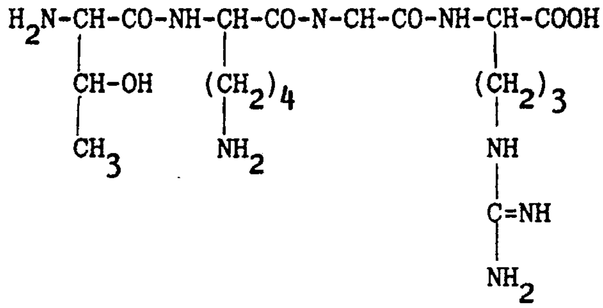

or of a tuftsin analogue of formula (II):

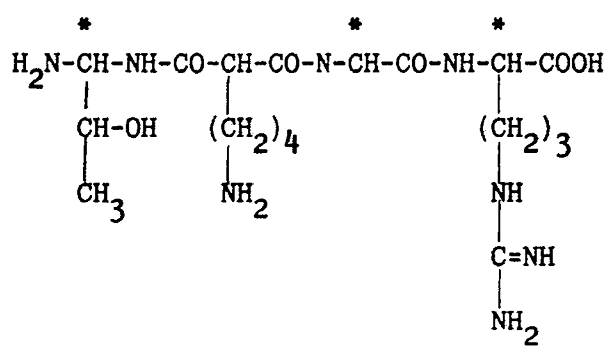

or a pharmaceutically acceptable salt of basic or acid addition thereof, as anti-inflammatory agents for the treatment of illnesses of anti-inflammatory pathogenesis, and to pharmaceutical compositions comprising a therapeutically effective quantity of the compound of formula (I) or formula (II) or of their salts of addition.

Inflammation is the positive response of an organism to pathogenic agents of chemical, physical, bacterial or viral nature, and is generally resolved by removing the pathogenic agent and healing the inflamed tissue.

However in some cases the inflammatory process becomes chronic. Even if the acute or chronic inflammatory transition mechanism is still not completely understood, it seems that in this process the activation of the immune system plays a fundamental role.

In this respect, chronic inflammation is characterised by the presence of immunocompetent cells such as macrophages and lymphocytes in the inflammatory focus. Chronic inflammatory processes are at the root of important human pathologies such as rheumatoid arthritis, osteoarthritis, lupus erythematosus, collagenopathies and glomerulonephritis.

Technical and patent literature describes numerous anti-inflammatory substances pertaining to various classes of compounds (in this respect see Goodman and Gilman, "The Pharmacological Basis of Therapeutics", 7th Edition, Macmillan Publishing Company, New York and London, 1985, pp 674-715).

It has now been surprisingly found that compounds of formula (I):

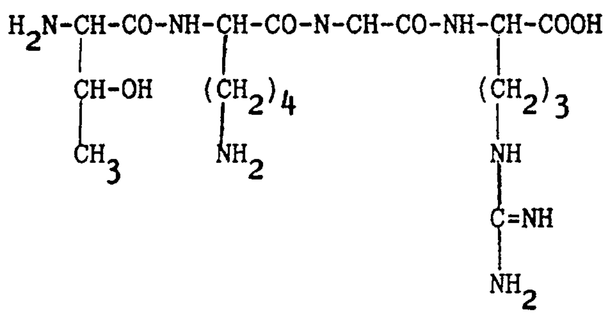

and of formula (II):

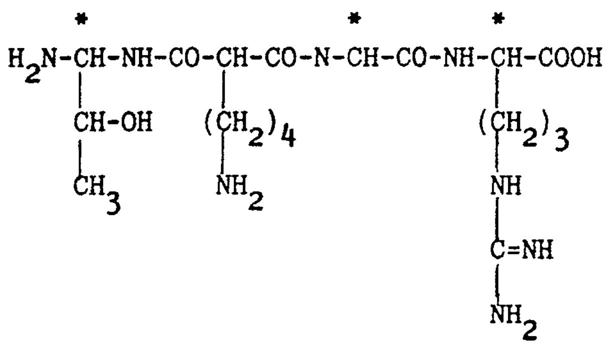

are, when administered to animals, able to demonstrate anti-inflammatory activity without inducing undesirable side effects. Therefore it is an object of the present invention to provide compounds of formula (I) and formula (II) for use as anti-inflammatory active principle in pharmaceutical compositions.

A further object of the present invention is to provide a pharmaceutical composition comprising as active principle a therapeutically effective quantity of the compound of formula (I) or formula (II) and a pharmaceutically acceptable non-toxic sterile carrier.

An other aim of the the present invention is also to provide a method for reducing inflammation in a subject or in an inflamed tissue, comprising administering to said subject, or bringing the inflamed tissue into contact with, an anti-inflammatory quantity of the compound of formula (I) or of formula (II) either in a single dose or in several successive doses.

Further objects of the present invention will be apparent from the following description.

In the aforegoing formula (II), the configuration of the carbon atoms indicated by an asterisk is the L-configuration, whereas the configuration of the carbon atom of the malonyl residue can be the L- or D-configuration.

Specifically, the compound with which the experimental work was carried out is a mixture of the two diastereoisomers with L-configuration at the asymmetric carbon atoms of the first, third and fourth amino acid residue or pseudo amino acid residue of the sequence, starting from the amino terminal residue (the atoms carrying the asterisk), and with D- or L- configuration at the asymmetric carbon atom of the malonyl residue.

The ccmpound of formula (II) is a retro-inverso analogue of natural tuftsin of formula (I) obtained as described in European patent application EP-253,190.

Tuftsin is a tetrapeptide of sequence Thr-Lys-Pro-Arg isolated in 1970 by a group of American researchers. It is detached from a particular immunoglobulin, leukoquinine, by the action of two enzymes, namely tuftsin-endocarboxypeptidase, a spleen enzyme which acts on the circulating leukoquinine to split the Arg-Glu linkage and thus releasing the tuftsin carboxyl termination, and leukoquininase, a membrane enzyme of neutrophils, monocytes and macrophages (MO), which cleaves the Thr-Lys linkage to thus release the tuftsin amino termination.

The main biological effect of tuftsin is to potentiate many functions of tissue macrophages and polymorphonucleate granulocytes, exhibiting its activity only when released from the leukoquinine carrier molecule.

As macrophages, besides being phagocytic cells, also play an essential role as accessory cells, i.e. cells employed in presenting the antigen, it is therefore likely that tuftsin can exercise a modulating activity

on chronic inflammation.

Tuftsin binds to the specific receptors present on the periplasmatic membrane of phagocytic cells, then passes through the membrane and into the cytoplasm where it is metabolized by certain enzymes of the cytoplasm. The most active of these is an aminopeptidase which detaches the threonine residue to form the tripeptide Lys-Pro-Arg, which is a potent inhibitor of tuftsin activity.

As described in the cited European patent application, the compound of formula (II), which can be indicated briefly in accordance with an internationally recognized terminology as:

gThr-mLys-Pro-Arg-OH

where "g" indicates that the amino acid residue Thr has been converted into the corresponding gem-alkyl-diamino derivative, and "m" indicates that the amino acid residue Lys has been converted into the corresponding malonyl derivative, has proved to possess substantially the same pharmacological activities as natural tuftsin (I) but with a greater stability towards plasma peptidase.

In accordance with the present invention the anti-inflammatory activity of the compounds of formula (I) and (II) was determined using an experimental model, namely adjuvant-induced arthritis in the rat (Turner R.A. "Screening Methods in Pharmacology", Academic Press, New York and London, 1965 pp. 152-163).

In practice the trial consists of injecting into the left rear paw of consanguineous Lewis rats 0.2 ml of complete Freund's adjuvant containing 10 mg/ml of Mycobacterium tuberculosis. After about 2 weeks an inflammatory process (secondary lesion) develops in the right rear paw and is quantified by measuring the paw volume using a water plethysmometer. The appearance of the inflammatory lesion in the uninjected paw is caused by activation of the animal immune system following administration of the adjuvant.

The anti-inflammatory effect of the compounds according to the present invention, which is manifested as a reduction in the volume of the inflamed paw, was studied by administering different doses of said compounds orally and parenterally (intraperitoneal and subcutaneous).

The results obtained showed for the first time anti-inflammatory activity both of natural tuftsin and of its retro-inverso analogue. It was also found that retro-inverted tuftsin is at least 10 times more effective than natural tuftsin when administered orally.

The present invention therefore enables the formulation of pharmaceutical compositions which can be used for therapeutic purposes at the effective doses in illnesses of inflammatory pathogenesis.

The compound of formula (I) or of formula (II) and their pharmaceutically acceptable salts of acid or basic addition can be used as anti-inflammatory active principle in the formulation of pharmaceutical compositions for the preparation of dosage forms suitable for oral, parenteral or topical administration.

The quantity of anti-inflammatory compound of formula (I) or (II) contained in a pharmaceutical composition can vary depending on different parameters such as the nature of the composition, including the particular compound contained in it, and the method of administration.

In any event, the concentration of said compounds is generally between 0.1 and 10 mg per kg of body weight.

The compounds (I) and (II) according to the present invention can be formulated in unit dosage forms, depending on the method of administration, each unit dosage containing an amount of active principle comprised between 10 and 100 mg.

In addition to the compounds of formula (I) or (II) as active principle, the formulations according to the present invention can contain stabilizers, adjuvants, preservatives, antioxidants, emulsifiers, anti-flocculants, buffers and disgregation agents chosen from those which are compatible with the compounds themselves and pharmacologically acceptable.

In the case of oral formulations, said pharmaceutical compositions can also contain flavourings, as known in the pharmaceutical field, all formulated in gastro-resistant dosage forms, typically gastro-resistant capsules, to prevent denaturation and enzymatic degradation of the active principle at the stomach level. The formulations according to the present invention can be prepared by mixing up the active principle/s with the pharmaceutically acceptable additives requested by the chosen method of administration according to the known procedures.

For example for formulations for oral administration (for example tablets) the peptides, in solid form, can be mixed up with the pharmaceutically acceptable diluting powders in an ordinary mixer, in order to obtain the wanted weight and volume necessary for allowing the requested compression; to the so obtained mixture the requested pharmaceutically acceptable additives can be added before the compression-phase.

In the case of formulations in the form of capsule, the peptide, as powder, can be introduced as such in the capsule and the requested additives can be added before the closure of the capsule.

Brief description of Figure 1

This shows the effect of different treatments on the development of the secondary lesion in adjuvant-induced arthritis in the rat. The horizontal axis represents days of treatment starting from day 0, on which complete Freund's adjuvant (CFA) is injected. The vertical axis represents the volume increase of the uninjected paw. The significance was calculated using Student's test for non-paired data.

The purpose of the following example is merely to better illustrate the present invention and must not be interpreted as implying any limitation thereto.

EXAMPLE 1

Effect of natural tuftsin and retro-inverted tuftsin on adjuvant-induced arthritis in the rat

The ability of the compounds of formula (I) and formula (II) (obtained as described in Example 1 of European patent application EP-A-253190) to exercise anti-inflammatory activity was evaluated by the experimental model described by Turner R.A. "Screening Methods in Pharmacology", Academic Press, New York and London, 1965 pp. 152-163.

The method enables the effect of drugs on chronic inflammation to be studied in an experimental model such as adjuvant-induced arthritis in the rat.

The model consists in practice of injecting into the left rear paw of consanguineous Lewis rats 0.2 ml of complete Freund's adjuvant containing 10 mg/ml of Mycobacterium tuberculosis. After about 2 weeks an inflammatory process (secondary lesion) develops in the right rear paw and is quantified by measuring the paw volume using a water plethysmometer. The appearance of the inflammatory lesion in the uninjected paw is caused by activation of the animal immune system following administration of the adjuvant.

a) Determination of anti-inflammatory activity of compounds of formula (I) and (II) administered orally

Groups of eight consanguineous Lewis rats of approximately 200-220 g body weight were treated parenterally on day 0 with 0.2 ml of complete Freund's adjuvant containing 10 mg/ml of Mycobacterium tuberculosis.

Starting from day 0, 0.2 ml of apyrogenic saline solution, either alone or containing 1 mg/kg of body weight of natural tuftsin (T-N) or 1 and 0.1 mg/kg of body weight of retro-inverted tuftsin (R-T), were administered orally (intragastric intubation) three times per week until the 38th day.

Starting from the 14th day the uninjected paw volume was measured using a water plethysmometer (Messrs. U. Basile, Varese, Italy).

The results, shown in Table I and Figure 1, indicate considerable anti-inflammatory activity both of T-N and of R-T (0.1 mg/kg).

TABLE I

Effect of oral pharmacological treatment on the development of inflammation in the uninjected paw (secondary lesion)

| Group | | % reduction | P |
|---|---|---|---|
| T-N | 1 mg/kg oral adm. | 23.3 | < 0.05 |
| R-T | 1 mg/kg oral adm. | 4.8 | n.s. |
| R-T | 0.1 mg/kg oral adm. | 36.7 | < 0.001 |

The data are expressed as mean percentage reduction with respect to the control group (not treated pharmacologically) calculated on the basis of paw volumes measured between the 14th and 38th day. Significance was calculated by Student's t test. n.s. = not significant.

b) Determination of anti-inflammatory activity of the compound of formula (II) administered intraperitoneally and subcutaneously

Groups of eight consanguineous Lewis rats of approximately 200-220 g body weight were treated parenterally on day 0 with 0.2 ml of complete Freund's adjuvant containing 10 mg/ml of Mycobacterium tuberculosis.

Starting from day 0, 0.2 ml of apyrogenic saline solution, either alone or containing 0.1 mg/kg of body weight of retro-inverted tuftsin (R-T), were administered intraperitoneally (i.p.) or subcutaneously (s.c.) into the back at the base of the neck, three times per week until the 38th day.

Starting from the 14th day the uninjected paw volume was measured using a water plethysmometer (Messrs. U. Basile, Varese, Italy). The results, shown in Table II and Figure 1, indicate that for the group treated by i.p. administration there is an appreciable reduction in secondary lesion development, which is statistically significant only during the initial days of treatment. The group of rats treated with R-T by s.c. administration shows no statistically significant reduction in secondary lesion.

TABLE II

Effect of parenteral pharmacological treatment on the development of inflammation in the uninjected paw (secondary lesion)

| Group | % reduction | P |
|---|---|---|
| R-T 0.1 mg/kg by i.p. adm. | 16.7 | n.s. |
| R-T 0.1 mg/kg by s.c. adm. | 2.4 | n.s. |

The data are expressed as mean percentage reduction with respect to the control group (not treated pharmacologically) calculated on the basis of paw volumes measured between the 14th and 38th day. Significance was calculated by Student's t test.
n.s. = not significant.

SEQ ID N°: 1

Lenght of the sequence : 4

Type of the sequence : amino acids

ThrLysProArg
1

SEQ ID N° : 2

Lenght of the Sequence : 4

Type of the sequence : amino acids

XaaX'aaProArg
1

Wherein : Xaa = gThr (corresponding Thr gem-alkyl-diamino derivative)

X'aa = mLys (corresponding Lys malonil derivative)

## Claims

1. A compound definable by the formula (I):

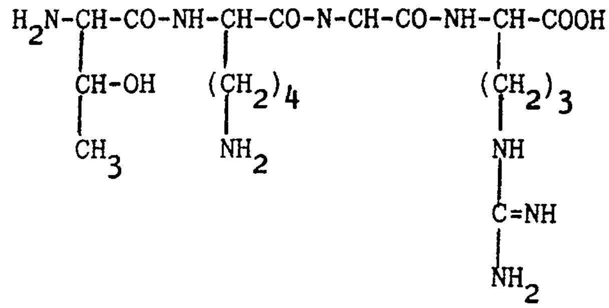

or by the formula (II):

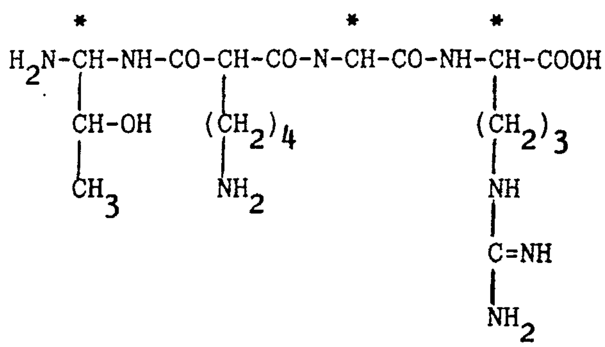

wherein the absolute configuration of the carbon atoms indicated by an asterisk is the L- configuration, and the absolute configuration of the carbon atom of the malonyl residue is the L- or D-configuration, or a pharmaceutically acceptable salt of basic or acid addition thereof, for use as an anti-inflammatory active principle in the preparation of pharmaceutical compositions.

2. A pharmaceutical composition comprising a therapeutically effective quantity of a compound claimed in claim 1.

3. A pharmaceutical composition as claimed in claim 2, wherein the compound of formula (II) is a mixture of diastereoisomers having an absolute L- configuration at the carbon atoms indicated by an asterisk and an L- or D- configuration at the asymmetric carbon atom of the malonyl residue.

4. A pharmaceutical composition as claimed in claims 2 and 3, comprising a quantity of compounds of formula (I) and (II) which is therapeutically effective in mammals including man, plus excipients and additives suitable for the various formulations.

5. A pharmaceutical composition according to any of the preeceding claims for the preparation of a single-dose administration unit containing from 10 to 100 mg of active principle.

6. Dosage form for oral administration of a pharmaceutical composition according to claims 1-4 containing from 10 to 100 mg of active principle.

7. Dosage form for parenteral administration of a pharmaceutical composition according to claims 1-4 containing from 10 to 100 mg of active principle.

**CLAIMS FOR THE FOLLOWING CONTRACTING STATES: GR AND ES**

1. A process for preparing pharmaceutical compositions for use as anti-inflammatory, containing as active

principle a peptide of formula (I)

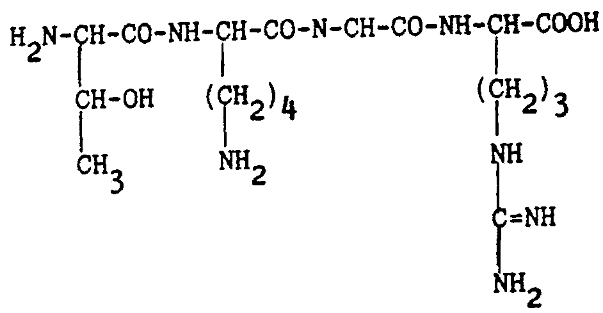

and/or a peptide of formula (II)

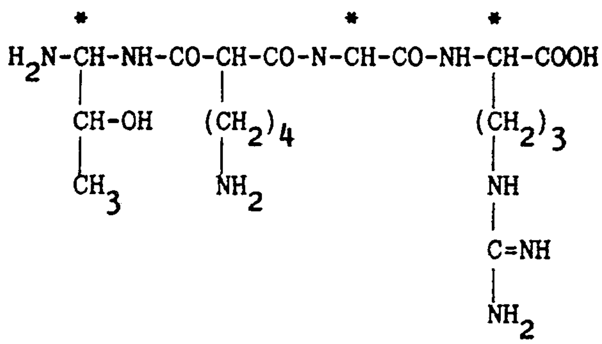

or their pharmaceutically acceptable salts, wherein the peptide, in solid form, is mixed up with the pharmaceutically acceptable diluting additives in an ordinary mixer; and to the so obtained mixture the other eventually requested pharmaceutically acceptable additives can be added.

2. Process according to claim 1, wherein the active principle is a peptide of formula (II) as mixture of diastereoisomers having an absolute L-configuration at the carbon atoms indicated by an asterisk and an L- or D-configuration at the asymmetric carbon atom of the malonyl residue.

3. A process according to claims 1 and 2, wherein the active principle/s present in an unity dosage form is comprised between 10 and 100 mg.

FIG.1

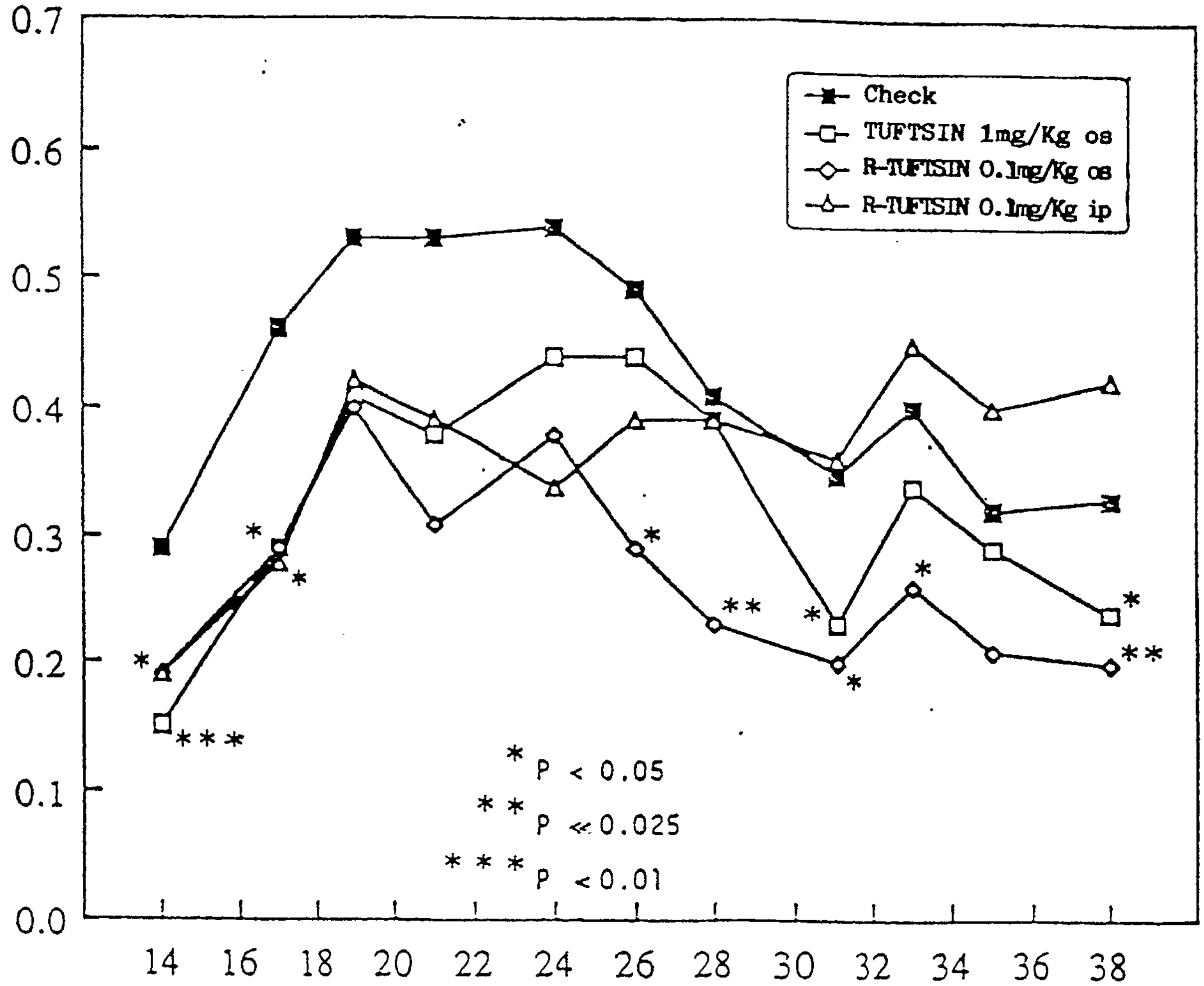
0.7
0.6
0.5
0.4
0.3
0.2
0.1
0.0
Check
TUFTSIN 1mg/Kg os
R-TUFTSIN 0.1mg/Kg os
R-TUFTSIN 0.1mg/Kg ip
* P < 0.05
* * P ≪ 0.025
* * * P < 0.01
14 16 18 20 22 24 26 28 30 32 34 36 38

European Patent Office

# EUROPEAN SEARCH REPORT

Application Number

EP 90124579.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP - A2/A3 - 0 253 190 (ENIRICERCHE) * Claims 1-5,8; page 7, line 55 - page 8, line 9 * -- | 1,2, 4-7 | C 07 K 5/00<br>A 61 K 37/02 |
| Y | WO - A1 - 84/00 108 (INSTITUT PASTEUR DE LILLE) * Claims 1,5,8; page 2, lines 19-24; page 4, lines 14-19; page 7, lines 14-23 * ---- | 1,2, 4-7 | |

TECHNICAL FIELDS SEARCHED (Int. Cl.5)

C 07 K
A 61 K

The present search report has been drawn up for all claims

| Place of search | Date of completion of the search | Examiner |
|---|---|---|
| VIENNA | 29-03-1991 | BÖHM |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)